# EUROPEAN PATENT APPLICATION

(11) **EP 4 059 960 A1**
(43) Date of publication of application: **21.09.2022**
(21) Application number: 20886654.1
(22) Date of filing: 12.11.2020
(51) Int. Cl.: C07K 16/18, C12N 5/0735, C12N 5/09, C12N 5/10, C12Q 1/6837, C12Q 1/6841, C12Q 1/6844, C12Q 1/686, C12Q 1/6862, C12Q 1/6869, C12Q 1/6872, C12Q 1/6876

(54) **METHOD FOR DETECTING UNDIFFERENTIATED CELLS**

(30) Priority: 15.11.2019 JP 2019207002
(71) Applicant: Public University Corporation Yokohama City University, Yokohama-shi, Kanagawa 236-0027 (JP); EIKEN KAGAKU KABUSHIKI KAISHA, Tokyo 110-8408 (JP)
(72) Inventor: TANIGUCHI, Hideki, Yokohama-shi, Kanagawa 236-0004 (JP); SEKINE, Keisuke, Yokohama-shi, Kanagawa 236-0004 (JP); YASUI, Ryota, Shimotsuga-Gun, Tochigi 329-0114 (JP)
(74) Representative: Hoffmann Eitle
(86) International application number: PCT/JP2020/042209
(87) International publication number: WO 2021/095797

(57) **Abstract**

The present invention provides a marker gene capable of detecting the undifferentiated cells that remain or become included in a differentiated cell population. Undifferentiated cells present in a differentiated cell population are detected by using at least one gene selected from the group consisting of *LINC00678* and *PRDM14* as an undifferentiation marker. A method of detecting undifferentiated cells; a method of using the gene as an undifferentiation marker; and a kit for detecting undifferentiated cells. A method of selecting an undifferentiated cell clone is also provided.

## Description

### TECHNICAL FIELD

The present invention relates to a method for detecting undifferentiated cells.

### BACKGROUND ART

From the viewpoint of the risk of oncogenesis, it is extremely important to detect and evaluate how much of undifferentiated iPS cells remain or become included in differentiated cell populations for use in regenerative medicine. To date, the following methods have been reported as techniques for detecting and evaluating such remaining or inclusion of undifferentiated iPS cells. Briefly, a method in which iPS cell specific genes are detected by quantitative PCR (qPCR) (Non-Patent Document No. 1) and a method in which differentiated cells are re-cultured under undifferentiated cell maintenance conditions (Non-Patent Document No. 2) have been reported.

Among the conventional methods, the re-culturing method has an advantage of high accuracy since colonies are formed from undifferentiated iPS cells that have become included in differentiated cells, but it takes more than one week for detection. Therefore, the method using quantitative PCR is superior in that it can be implemented in a simple and quick manner.

However, with respect to *LIN28* reported previously (Non-Patent Document No. 1), while its expression is low in a differentiated cell of organ/tissue (retinal pigment epithelial cell), expression is observed in other differentiated cells (such as hepatic cells) and cells that result from directed differentiation of iPS cells. Therefore, *LIN28* cannot be used for detecting the remaining or inclusion of undifferentiated iPS cells in a wide range of differentiated cells.

### PRIOR ART LITERATURE

### Non-Patent Documents

Non-Patent Document No. 1: PLoS One. 2014 27;9(10):e110496
Non-Patent Document No. 2: PLoS One. 2012;7(5):e37342

### DISCLOSURE OF THE INVENTION

### PROBLEM FOR SOLUTION BY THE INVENTION

It is an object of the present invention to provide a marker gene by which even undifferentiated cells that remain or become included in differentiated cells other than retinal pigment epithelial cell can be detected.

### MEANS TO SOLVE THE PROBLEM

The present inventors have identified marker genes that are applicable to a wide variety of differentiated cells and that are capable of universal detection of the remaining or inclusion of undifferentiated iPS cells.

No marker gene is ideal if it satisfies only the following two requirements:
1. It is specifically expressed in undifferentiated iPS cells;
2. Its expression is extremely low in cell lineages other than undifferentiated iPS cells.
   An additional criterion to be met is the following requirement that must be satisfied to enable detection even when only a few undifferentiated cells are present in differentiated cells:
3. Its expression is extremely high in undifferentiated iPS cells.

Then, the present inventors have found, as genes that satisfy the above criteria, *LINC00678* and *PRDM14* which are expressed highly in undifferentiated iPS cells and whose expression becomes 0.1% or less in differentiated endodermal cells. By using these genes, it has become possible to detect the remaining or inclusion of undifferentiated iPS cells to as low as 0.025% in differentiated cells of organs/tissues. Further, since the expression of these marker genes also becomes 0.1% or less even in cells that have differentiated to mesodermal and ectodermal cells, it is believed that these genes are markers capable of detecting the remaining or inclusion of undifferentiated iPS cells in any one of differentiated endodermal, mesodermal or ectodermal cells.

A summary of the present invention is as described below.
(1) A method of detecting undifferentiated cells, comprising measuring the expression level and/or the promoter activity of at least one gene selected from the group consisting of *LINC00678* and *PRDM14* in a differentiated cell population.
(2) The method of (1) above, wherein the undifferentiated cell is embryonal carcinoma cell (EC cell), embryonic stem cell (ES cell), induced pluripotent stem cell (iPS cell) or embryonic germ cell (EG cell), and the differentiated cell population is a population of cells which have been differentiated from the undifferentiated cell.
(3) The method of (1) or (2) above, wherein the differentiated cell population is a population of differentiated endodermal, mesodermal or ectodermal cells.
(4) The method of (3) above, wherein the differentiated endodermal cells are hepatic endoderm cells.
(5) The method of any one of (1) to (4) above, wherein the expression level of the gene is measured as the amount of RNA containing mRNA or the amount of protein.
(6) The method of any one of (1) to (5) above, wherein the expression level of the gene is measured by qPCR, digital PCR, isothermal amplification of nucleic acids, immunostaining, *in situ* hybridization, RNA sequencing, microarray, NanoString, antibody array, flow cytometry or mass spectrometry.
(7) A method of selecting a highly safe undifferentiated cell clone, comprising measuring the expression level and/or the promoter activity of at least one gene selected from the group consisting of *LINC00678* and *PRDM14* in differentiated endodermal, mesodermal or ectodermal cells that result from directed differentiation of an undifferentiated cell clone.
(8) The method of (7) above, wherein the undifferentiated cell clone is an embryonal carcinoma cell (EC cell) clone, an embryonic stem cell (ES cell) clone, an induced pluripotent stem cell (iPS cell or iPSC) clone or an embryonic germ cell (EG cell) clone.
(9) The method of (8) above, wherein the undifferentiated cell clone is an iPS cell clone.
(10) A method of detecting undifferentiated cells, comprising measuring the expression level and/or the promoter activity of at least one gene selected from the group consisting of *LINC00678* and *PRDM14* in a tissue formed by transplanting differentiated cells into a model animal.
(11) A method of using at least one gene selected from the group consisting of *LINC00678* and *PRDM14* as an undifferentiation marker for detecting undifferentiated cells in a differentiated cell population.
(12) A kit for detecting undifferentiated cells, comprising a reagent capable of detecting the expression of at least one gene selected from the group consisting of *LINC00678* and *PRDM14* and/or a reagent capable of measuring the promoter activity of the gene.
(13) The kit of (12) above, wherein the reagent capable of detecting the expression of the gene is primers, probes or antibodies.
(14) The kit of (12) above, wherein the reagent capable of measuring the promoter activity of the gene is a gene sequence in which a reporter protein is ligated downstream of the promoter or a vector incorporating the gene sequence.

### EFFECT OF THE INVENTION

According to the present invention, it is possible to detect and evaluate how much of undifferentiated cells remain or become included in a differentiated cell population and this contributes to reducing the risk of oncogenesis in differentiated cells for use in regenerative medicine.

The present specification encompasses the contents disclosed in the specification and/or drawings of Japanese Patent Application No. 2019-207002 based on which the present patent application claims priority.

### BRIEF DESCRIPTION OF THE DRAWINGS

[Fig. 1] Mouse *LIN28A* expression in hepatic cells during liver development (microarray data). Compared to adult (8w), expression is high until E13.5.
[Fig. 2] *LIN28A* expression at respective differentiation stages of directed differentiation from human iPSCs to hepatic cells. *LIN28A* expression is also high in definitive endoderm (DE) and hepatic endoderm (HE).
[Fig. 3] qPCR of genes extracted by microarray. Upon confirmation of expression by qPCR, genes whose expression is especially low in hepatic cells were extracted.
[Fig. 4] Examination of detection sensitivity of marker genes by undifferentiated iPSC spike-in experiments. Undifferentiated iPSCs were mixed into hepatic endoderm cells. The resultant experiment groups were compared to control group without spiking of undifferentiated iPSCs, to thereby examine detection sensitivity by qPCR. ^{∗}p<0.05.
[Fig. 5] iPS cells underwent directed differentiation to individual cells derived from three germ layers using STEMdiff^{™} Trilineage Differentiation Kit (STEMCELL Technologies). Then, it was confirmed by immunostaining and qPCR that directed differentiation was performed successfully.
[Fig. 6] Expression of marker genes in the cells resulting from directed differentiation in Fig. 5 was examined by qPCR.
[Fig. 7] Residual undifferentiated cell counts in the cells resulting from directed differentiation in Fig. 5 was evaluated by colony immunostaining.
[Fig. 8] RT-LAMP reaction composition.
[Fig. 9] Examination of marker gene detection sensitivity of RT-LAMP by undifferentiated iPSC spike-in experiments.
[Fig. 10] Expression of undifferentiation markers and evaluation of residual iPSC in vascular endothelial cells resulting from directed differentiation of iPS cells (iPSC-EC). Differentiation to vascular endothelial cells was confirmed by expression of *CD34* and *CDH5.* Expression of undifferentiation detection marker genes (*ESRG, LINC00678* and *PRDM14*) was examined by qPCR.
[Fig. 11] Expression of undifferentiation markers and evaluation of residual iPSC in mesenchymal cells that result from directed differentiation of iPS cells (iPSC-STM/MC). Differentiation to mesenchymal cells was confirmed by expression of *FOXF1* and *PDGFRB.* Expression of undifferentiation detection marker genes (*ESRG, LINC00678* and *PRDM14*) was examined by qPCR.
[Fig. 12] Expression of undifferentiation markers and evaluation of residual iPSC in ectodermal cells that result from directed differentiation of iPS cells (neural crest cells: NCC). Differentiation to neural crest cells was confirmed by expression of *SOX1* and *PAX6.* Expression of undifferentiation detection marker genes (*ESRG, LINC00678* and *PRDM14*) was examined by qPCR.

### DESCRIPTION OF EMBODIMENTS FOR CARRYING OUT THE INVENTION

Hereinbelow, the present invention will be described in detail.

The present invention provides a method of detecting undifferentiated cells, comprising measuring the expression level and/or the promoter activity of at least one gene selected from the group consisting of *LINC00678* (HUGO Gene Nomenclature Committee (HGNC) Official Full Name: long intergenic non-protein coding RNA 678; NCBI Reference Sequence: NR_102708.1, etc.) and *PRDM14* (HGNC Official Full Name: PR/SET domain 14; NCBI Reference Sequence: NM_024504.4, etc.) in a differentiated cell population.

The undifferentiated cell that is the target of detection may be a cell with pluripotency, e.g., embryonal carcinoma cell (EC cell), embryonic stem cell (ES cell), induced pluripotent stem cell (iPS cell) or embryonic germ cell (EG cell).

Cells constituting the differentiated cell population may be any cells other than undifferentiated cells and, preferably, cells without pluripotency. For example, cells constituting the differentiated cell population are those cells which have been differentiated from an undifferentiated cell that is the target of detection.

The differentiated cell population may be a population of any one of endodermal, mesodermal or ectodermal differentiated cells.

Examples of endodermal differentiated cells include, but are not limited to, hepatic endoderm cells.

In one Example described later, the present inventors detected iPS cells (undifferentiated cells) that were caused to become included in a population of hepatic endoderm cells (differentiated cells) resulting from directed differentiation of iPS cells. These hepatic endoderm cells are hepatic progenitor cells designated iPSC-HE (hepatic endoderm) at day 10 of directed differentiation treatment from iPS cells to hepatic cells (Nature 499:481-484 (2013); Japanese Patent No. 6124348 "Method of Preparing Tissues and Organs"). According to measurement by qPCR, it is believed that *LINC00678* and *PRDM14* are effective marker genes for detecting iPS cells in hepatic endoderm cell populations.

Examples of mesodermal differentiated cells include, but are not limited to, *septum transversum* mesenchyme cells, mesenchymal cells and vascular endothelial cells.

In one Example described later, the present inventors detected iPS cells (undifferentiated cells) that were caused to become included in a population of mesenchymal cells (differentiated cells) resulting from directed differentiation of iPS cells. These mesenchymal cells are mesenchymal stem/progenitor cells (mesoderm-derived cells) designated iPSC-STM/MC [iPS cell-derived *septum transversum* mesenchyme cells/iPS cell-derived mesenchymal cells (septum transversum mesenchyme/mesenchymal cells)] (Cell Rep. 21:2661-2670, 2017) which received directed differentiation treatment from iPS cells to mesenchymal cells. These mesenchymal cells are CD166 positive and do not express *CD31 (PECAM1),* a vascular endothelial marker. According to measurement by qPCR, it is believed that *LINC00678* and *PRDM14* are effective marker genes for detecting iPS cells in mesenchymal cell populations.

Further, in one Example described later, the present inventors detected iPS cells (undifferentiated cells) that were caused to become included in a population of vascular endothelial cells (differentiated cells) resulting from directed differentiation of iPS cells. These vascular endothelial cells are endothelial progenitor cells (mesoderm-derived cells) designated iPSC-EC (iPS cell-derived endothelial cell) (Cell Rep. 21:2661-2670 (2017)) which received directed differentiation treatment from iPS cells to vascular endothelial cells. In these vascular endothelial cells, expression of the proteins of vascular endothelial markers CD31 (PECAM1) and CD144 can be confirmed by immunostaining. In gene expression analyses, high expression of vascular endothelial markers such as *PECAM1, CDH5, KDR, CD34* and the like is observed; compared to iPS cells before directed differentiation, 10- to more than 100-fold higher expression is observed in these vascular endothelial cells. According to measurement by qPCR, it is believed that *LINC00678* and *PRDM14* are effective marker genes for detecting iPS cells in vascular endothelial cell populations.

Examples of ectodermal differentiated cells include, but are not limited to, neural stem cells, neural crest cells and neural cells.

In one Example described later, the present inventors detected iPS cells (undifferentiated cells) that were caused to become included in a population of neural crest cells resulting from directed differentiation of iPS cells (Menendez L. et al., Proc Natl Acad Sci U S A. 108(48):19240-5. 2011) (ectoderm-derived cells). According to measurement by qPCR, it is considered that *LINC00678* and *PRDM14* are effective marker genes for detecting iPS cells in neural crest cell populations.

Further, in one Example described later, expression of marker genes was also detected by qPCR in three individual germ layer-derived cells that were obtained by directed differentiation using STEMdiff^{™} Trilineage Differentiation Kit (STEMCELL Technologies).

In the present invention, differentiated cells and undifferentiated cells may be derived from human or any of non-human animals.

The expression level of a marker gene may be measured as the amount of the RNA containing mRNA transcribed from the gene or the amount of the protein translated from the RNA containing mRNA. Specifically, the expression level of the gene may be measured by qPCR, digital PCR, isothermal amplification of nucleic acids (LAMP (Loop-Mediated Isothermal Amplification), etc.), immunostaining, *in situ* hybridization, RNA sequencing, microarray, NanoString, antibody array, flow cytometry, mass spectrometry or the like. It should be noted here that the RNA containing mRNA may be one which does not encode a protein. Alternatively, the RNA containing mRNA may be a partial degradation product or the like of an RNA comprising a target sequence of nucleic acid amplification.

When expression of at least one gene selected from the group consisting of *LINC00678* and *PRDM14* is confirmed, it is possible to judge that undifferentiated cells are present in the differentiated cell population of interest (detection of undifferentiated cells).

In the present specification, the term "detection" means confirmation of presence, but this term also encompasses confirmation of absence.

According to the method of the present invention, it is possible to detect undifferentiated cells in a differentiated cell population at a detection sensitivity of 0.1% or less, e.g., 0.025%, 0.01% or even 0.005% or 0.0025%. Detection sensitivity can be examined by the spike-in experiment described in an Example provided later.

It also becomes possible to select a highly safe undifferentiated cell clone by measuring the expression level and/or the promoter activity of at least one gene selected from the group consisting of *LINC00678* and *PRDM14* in differentiated endodermal, mesodermal or ectodermal cells that result from directed differentiation of an undifferentiated cell clone. Therefore, the present invention provides a method of selecting a highly safe undifferentiated cell clone in which undifferentiated cells are difficult to remain after directed differentiation, comprising measuring the expression level and/or the promoter activity of at least one gene selected from the group consisting of *LINC00678* and *PRDM14* in any one of differentiated endodermal, mesodermal or ectodermal cells that result from directed differentiation of an undifferentiated cell clone.

The undifferentiated cell clone that is the target of selection may be a cell clone with pluripotency. For example, the undifferentiated cell clone may be embryonal carcinoma cell (EC cell) clone, embryonic stem cell (ES cell) clone, induced pluripotent stem cell (iPS cell or iPSC) clone or embryonic germ cell (EG cell) clone. Among them, iPS cell clone is preferable.

Further, the expression level and/or the promoter activity of at least one gene selected from the group consisting of *LINC00678* and *PRDM14* in a tissue formed by transplanting differentiated cells into a model animal may be measured to detect undifferentiated cells in the tissue. Therefore, the present invention also provides a method of detecting undifferentiated cells, comprising measuring the expression level and/or the promoter activity of at least one gene selected from the group consisting of *LINC00678* and *PRDM14* in a tissue formed by transplanting differentiated cells into a model animal. Suitably, the tissue may be one formed by transplanting differentiated cells into a model animal over a long period of time (e.g., 4 to 54 weeks, preferably 8 to 24 weeks).

According to the present invention, it has become clear that *LINC00678* and *PRDM14* can be used as marker genes for detecting undifferentiated cells present in a differentiated cell population. Therefore, the present invention provides a method of using at least one gene selected from the group consisting of *LINC00678* and *PRDM14* as an undifferentiation marker for detecting undifferentiated cells present in a differentiated cell population.

The present invention also provides a kit for detecting undifferentiated cells, comprising a reagent capable of detecting the expression of at least one gene selected from the group consisting of *LINC00678* and *PRDM14* and/or a reagent capable of measuring the promoter activity of the gene.

As the reagent capable of detecting the expression of the gene, primers, probes, antibodies or the like may be enumerated. Examples are: a set of oligonucleotide primers capable of specifically amplifying the transcription product (RNA containing mRNA) or cDNA of at least one gene selected from the group consisting of *LINC00678* and *PRDM14*; a nucleotide probe specifically hybridizing with the transcription product (RNA containing mRNA) or cDNA of at least one gene selected from the group consisting of *LINC00678* and *PRDM14*; and an antibody specifically binding to the protein (translation product) translated from the transcription product (RNA containing mRNA) of at least one gene selected from the group consisting of *LINC00678* and *PRDM14.* The set of oligonucleotide primers may be a set of primers capable of amplifying a target sequence (usually, approximately 50-180 bp) in the nucleotide sequence of the transcription product (RNA containing mRNA) or cDNA of at least one gene selected from the group consisting of *LINC00678* and *PRDM14.* Such a set of primers may be so designed that they have sequences complementary to both ends of the target sequence. The length of oligonucleotide primers may, for example, be 15-35 nucleotides, preferably 18-27 nucleotides. The nucleotide probe may be one hybridizing with the transcription product (RNA containing mRNA) or cDNA of at least one gene selected from the group consisting of *LINC00678* and *PRDM14* under stringent conditions. The nucleotide probe may be so designed that it has a part or whole of the nucleotide sequence of the above RNA containing mRNA or cDNA, or a sequence complementary thereto. Stringent conditions may be appropriately selected. The length of nucleotide probes may be usually 1,000 nucleotides or less, preferably 100 nucleotides or less, more preferably 50 nucleotides or less, and still more preferably 5-30 nucleotides or 14-30 nucleotides. The nucleotide probe may be either single-stranded or double-stranded. The antibody may be either monoclonal or polyclonal. As used herein, the term "antibody" is a concept encompassing not only full-length antibodies but also antibodies of smaller molecular sizes such as Fab, F(ab)'₂, ScFv, Diabody, V_{H}, V_{L}, Sc(Fv)₂, Bispecific sc(Fv)₂, Minibody, ScFv-Fc monomer and ScFv-Fc dimer. Probes and antibodies may be immobilized on a solid phase (such as substrate, beads, membrane, etc.).

The reagent of the present invention may be labeled. For example, primers may be labeled with a fluorescent substance, a quencher, or the like; and probes and antibodies may be labeled with a radioactive isotope, an enzyme, a luminescent substance, a fluorescent substance, biotin or the like. In the case where a target molecule (which, in the present invention, is a protein as the expression product of at least one gene selected from the group consisting of *LINC00678* and *PRDM14)* is to be detected by first reacting a primary antibody that specifically binds to the target molecule and then reacting a secondary antibody that binds to the primary antibody, the secondary antibody may be labeled (the primary antibody is not labeled).

As the reagent capable of measuring the promoter activity of the gene, a gene sequence in which a reporter protein is ligated downstream of the promoter, a vector incorporating the gene sequence, or the like may be enumerated. Examples of the reporter protein include, but are not limited to, fluorescent proteins such as luciferase or GFP, and proteins such as CD antigen that are expressed in the cell membrane. As the vector, plasmid vectors are preferable.

The kit of the present invention may further comprise reagents for detecting genes with primers (e.g., DNA polymerase, buffer, magnesium ion, dNTPs, probe, etc.), reagents for detecting genes with probes (e.g., buffer, antibody, substrate, etc.), reagents for detecting genes with antibodies (e.g., secondary antibody, substrate, buffer, etc.), reagents for measuring the promoter activity of genes (e.g., buffer, luminescent substrate, antibody, etc.), instruments (reaction vessel, pipette, etc.), written instructions for using the kit, control samples, control data for analyzing measurement results, and so forth.

### EXAMPLES

Hereinbelow, the present invention will be described more specifically with reference to the following Examples. However, the scope of the present invention is not limited to these Examples.

### [Examples 1]

### Materials and Methods

### • iPSC

iPS cells kindly provided by Kyoto University and University of Tokyo were used (TkDA3-4, 1231A3, 1383D2, 1383D6 and Ff01).

### • Residual Undifferentiated Cells

In order to quantify undifferentiated cells remaining in the cells differentiated from iPSC, the present inventors applied the method described by Tano et al. Briefly, differentiated cells were detached with trypsin, seeded in ROCK inhibitor-containing StemFit in 24-well plates at 1.6 × 10⁵ cells/well, and cultured at 37°C with the culture medium being exchanged with StemFit every day. One week later, immunostaining was performed and positive colonies were counted.

### • Colony Counting

Immunostained samples were photographed with a microscope. Colonies on those photographs were counted visually. The number of residual undifferentiated iPS cells was based on the assumption that one colony of undifferentiated iPSC was derived from one undifferentiated iPS cell.

### • Hepatocyte Differentiation

Undifferentiated iPS cells were seeded on laminin-coated dishes in the presence of ROCK inhibitor (Y-27632) at a density of 5-10 × 10⁴ cells/cm². Cells were cultured for 6 days in the presence of RPMI+B27+activin A+Wnt3A to obtain definitive endoderm cells (DE). The resultant cells were further cultured for 4 days in the presence of KO-DMEM+KSR+DMSO+2-Mercaptoethanol to obtain hepatic endoderm cells (HE).

### • Immunostaining

In order to detect undifferentiated cells, the present inventors performed immunostaining using primary antibodies against pluripotency markers SOX2 and TRA-1-60 (Cell Signaling Technologies) and the corresponding secondary antibodies (Thermo Fisher Scientifics). Cells were fixed with 4% paraformaldehyde for 15 min, and washed twice with PBS. Cell membranes were permeabilized with 0.1% TritonX-100 in PBS (PBST) for 10 min and subsequently blocked with 5% FBS in PBST. After one hour, blocking buffer was removed. Appropriately diluted solution of the primary antibodies was added and cells were treated at 4°C overnight. Then, cells were washed three times with PBS. Diluted solution of the secondary antibodies was applied and the mixture was allowed to settle undisturbed for one hour at room temperature under light shielding conditions. In the last step, cells were washed three times with PBS, followed by addition of Apathy's Mounting Media (FUJIFILM Wako Pure Chemical Corporation) for observation.

### • Microscope (KEYENCE, etc.)

Whole-well images were obtained using an all-in-one fluorescence microscope BZ-X710 in bright field and blue, green and red fluorescence channels with objective lenses x4 and ×10.

### • qPCR

RNA containing mRNA was extracted from cells using PureLink RNA Mini Kit (Thermo Fisher) and cDNA was synthesized by reverse transcription using High-Capacity cDNA Reverse Transcription Kit (Thermo Fisher). Then, qPCR was performed using the following primers and Universal Probe Library (Roche). As regards *PRDM14,* respective primer pairs were designed for the total four regions of junction region between exons 1 and 2, junction region between exons 2 and 3, junction region between exons 4 and 5, and junction region between exons 6 and 7. Taking the following points into consideration, the present inventors designed such primer pairs: the transcription start site of RNA containing mRNA varies depending on the differentiation stage of cells in the process of directed differentiation from a pluripotent stem cell to a differentiated cell of interest; different exon-junction structures may occur as a result of splicing; partial degradation products of RNA may be produced and accumulated; and so forth. In other words, the present inventors considered that the sensitivity or accuracy in the detection of undifferentiated cells may vary depending on the position of designed primers.

**LINC00678**

| | |
|---|---|
| Forward: | catctcaccaattttaaatcaggac (SEQ ID NO: 1) |
| Reverse: | ctcccgtcattctgctaacac (SEQ ID NO: 2) |
| Probe: #17 | |

**PRDM14 E1-2**

| | |
|---|---|
| Forward: | gctcttggaggtggtgtcg (SEQ ID NO: 3) |
| Reverse: | gccggaaaggttggaagtc (SEQ ID NO: 4) |
| Probe: #64 | |

**PRDM14 E2-3**

| | |
|---|---|
| Forward: | tgcaccatgcgatttcag (SEQ ID NO: 5) |
| Reverse: | tgcatgaggcatagaccttc (SEQ ID NO: 6) |
| Probe: #79 | |

**PRDM14 E4-5**

| | |
|---|---|
| Forward: | gacaattctgtgatgtgggag (SEQ ID NO: 7) |
| Reverse: | tgacactgcacagcaactagg (SEQ ID NO: 8) |
| Probe: #68 | |

**PRDM14 E6-7**

| | |
|---|---|
| Forward: | ggcttcggatccacattc (SEQ ID NO: 9) |
| Reverse: | agtggactcgcatgtgtttg (SEQ ID NO: 10) |
| Probe: #11 | |

### • Spike-In Experiments

iPS cells cultured under conditions for maintaining undifferentiated state were caused to become included in cells resulting from directed differentiation at the proportions indicated in the Figures. Using the resultant cell mixtures, a test for residual undifferentiated cells, qPCR, gel electrophoresis of amplified products from qPCR, etc. were performed.

### • Statistics

P values <0.05 were considered significant by Student's t-test. Correlation coefficients were calculated based on Pearson product-moment correlation coefficient.

### Results

### ▪ LIN28 is not useful as an indicator for undifferentiated iPSC in the liver.

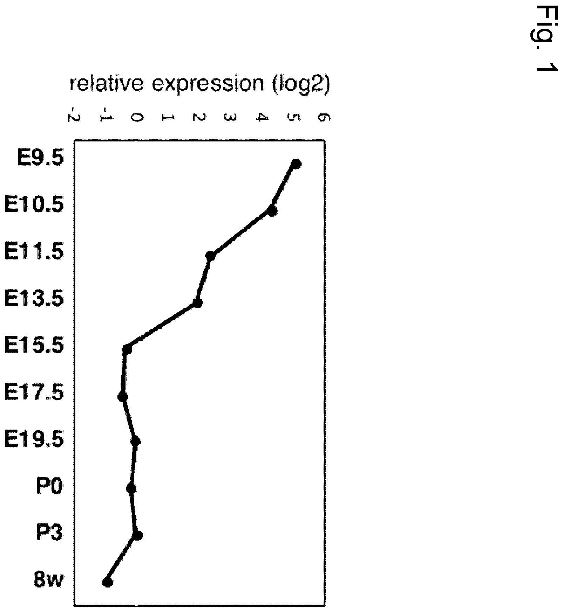

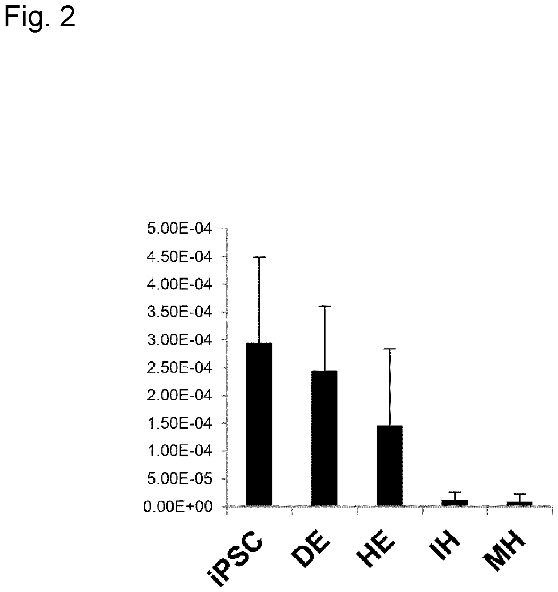

It has been reported that *LIN28* (*LIN28A*) is useful as an indicator for residual undifferentiated iPSC in retinal pigment epithelial cells (RPE) resulting from directed differentiation of iPSC (Kuroda T. et al., PLoS ONE 7(5):e37342.(2012)). When *LIN28* expression was observed in mouse liver during development, it became clear that *LIN28* expression was high until E13.5 compared to the expression in adult (8w) (see Fig. 1). It also became clear that in iPS cell-derived endodermal cells, i.e., definitive endoderm cells (DE) and hepatic endoderm cells (HE), *LIN28* expression hardly decreased as compared to the expression in undifferentiated iPS cells (see Fig. 2).

### ▪ Extraction of genes that are specifically expressed in iPS cells with high yield

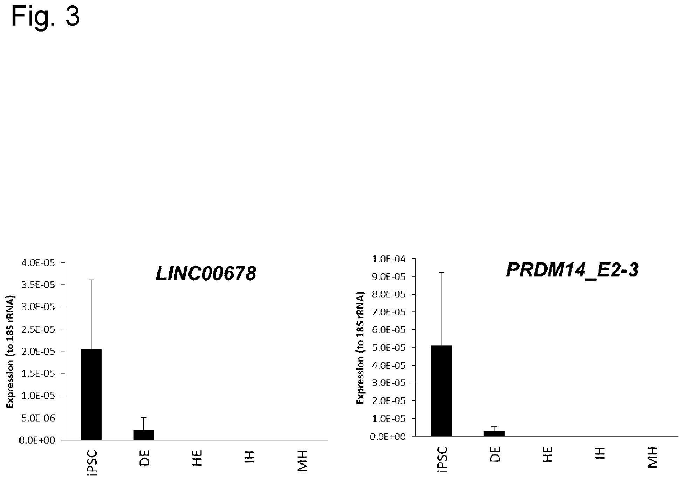

In order to extract marker genes as indicators for residual undifferentiated iPSC that might be useful in iPS cell-derived hepatic cells, the present inventors performed microarray analyses of mouse developmental stages, single cell RNA sequence analyses during processes of directed differentiation of iPS cell-derived hepatic cells, and sashimi plot analysis based on RNA sequence data, whereby more than 30 genes were extracted that were expressed in undifferentiated iPS cells both specifically and with high yield but whose expression was low in differentiated cells. By subjecting the extracted genes to qPCR, *LINC00678* and *PRDM14* were extracted that were expressed in iPS cells with high yield but whose expression decreased in differentiated cells (Fig. 3). Even in candidate genes that had been considered promising upon transcriptome analysis, most of them had problems such as low expression in iPS cells or high expression in differentiated cells, when examined by qPCR. Thus, those candidate genes were not useful markers.

### ▪ Examination of detection limits of respective marker genes (undifferentiated iPSC spike-in experiments)

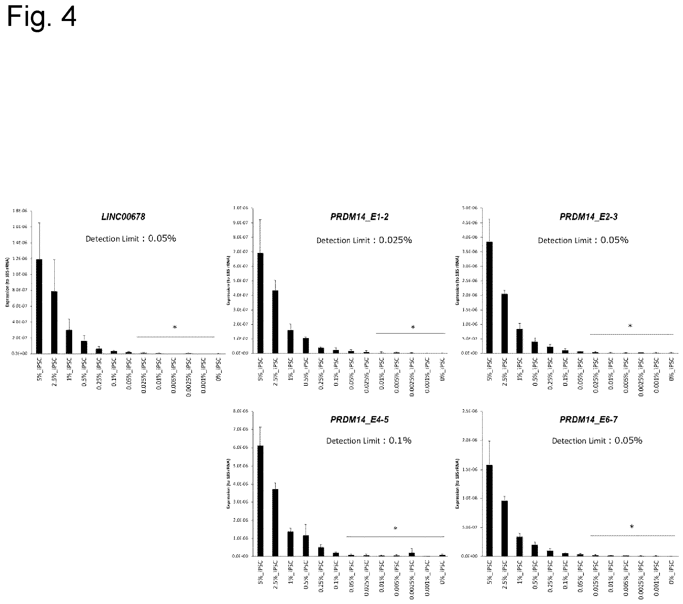

iPS cells cultured under conditions for maintaining undifferentiated state were caused to become included in iPS-cell derived hepatic progenitor cells (HE) resulting from directed differentiation at the proportions indicated in the Figures, and qPCR was performed on the resultant cell mixtures (Fig. 4). As a result, the remaining or inclusion of undifferentiated iPS cells was detectable up to 0.025%.

### ▪ Residual undifferentiated cell experiments in differentiated cells prepared with STEMdiff^{™} Trilineage Differentiation Kit

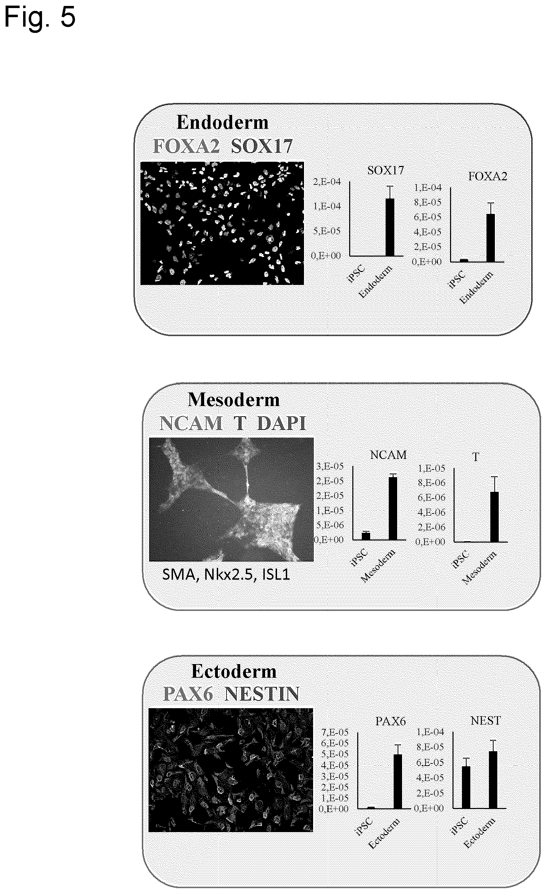

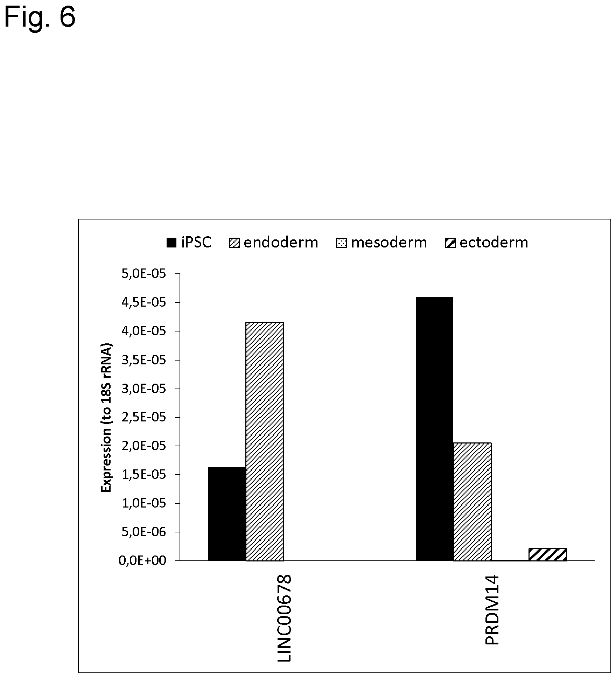

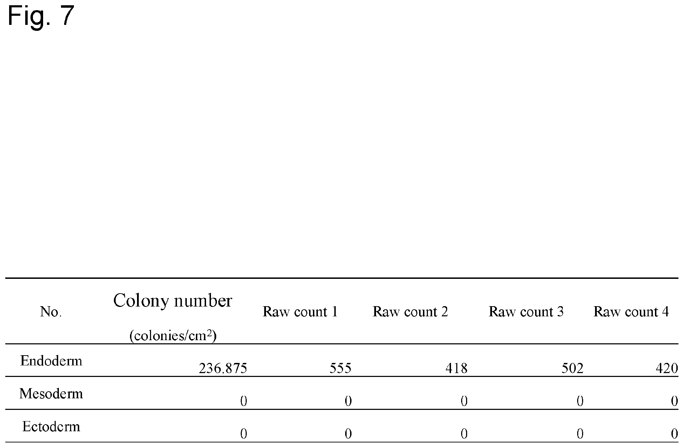

Further, in order to show that the above genes are generally useful as markers for residual undifferentiated cells in iPS cell-derived cells resulting from directed differentiation, the present inventors performed examination using cells that resulted from directed differentiation with a commercial directed differentiation kit (STEMdiff^{™} Trilineage Differentiation Kit; STEMCELL Technologies) (Fig. 5). As a result, residual undifferentiated cells were observed in endoderm-derived cells resulting from directed differentiation of iPS cells but at the same time, marker gene expression was high in correlation with the number of residual undifferentiated cells. Therefore, correlation between the number of residual undifferentiated cells and marker expression was observed in any of the cell lineages resulting from directed differentiation of iPS cells, i.e., endoderm-derived cells, mesoderm-derived cells and ectoderm-derived cells (Figs. 6 and 7).

### Discussion

Detection and exclusion of undifferentiated cell contamination in iPS (ES) cell-derived differentiated cells which contribute to applications in regenerative medicine are two important issues in ensuring the safety of all products processed from iPS (ES) cell-derived cells. To date, rapid evaluation of undifferentiated cell contamination by verification of *LIN28A* expression in retinal pigment epithelial cells (RPE) has been reported. However, *LIN28A* is expressed in the liver during mouse development. As a matter of fact, expression of *LIN28A* is also observed in cells resulting from directed differentiation of human iPS cells, and it became clear that this expression did not correlate with the presence or absence of undifferentiated cells actually remaining in differentiated cells. Accordingly, the present inventors extracted *LINC00678* and *PRDM14* as markers for residual undifferentiated cells in iPS cell-derived hepatic cells. The technique for detecting residual undifferentiated iPS cells using the plurality of marker genes as extracted in accordance with the present invention is expected to provide a simple and quick tool for ensuring the safety of various products processed from iPS (ES) cell-derived cells.

### References:

▪ Kuroda T. et al., PLoS ONE 7(5):e37342 (2012)
▪ Tano et al., PLoS One. 2014 27;9(10):e110496

### [Example 2]

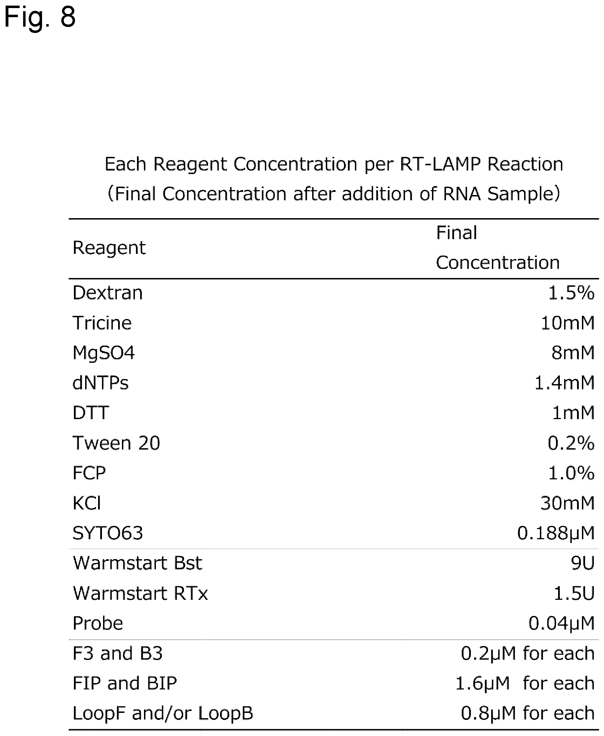

Isothermal amplification of nucleic acids was examined as a method for measuring the expression level of the marker gene of the present invention. As the isothermal amplification technique, LAMP method was used. Briefly, RT-LAMP method which amplifies and detects *LINC00678*-derived RNA structures was designed. Nucleotide sequences of the primer sets and the probe designed for the RT-LAMP are as described below. The probe was fluorescently labeled. Reaction composition for RT-LAMP is shown in Fig. 8.

| | |
|---|---|
| F3: | gacgggagtgtgagatcc (SEQ ID NO: 11) |
| B3: | acatcttctcctgaatcctcag (SEQ ID NO: 12) |
| FIP: | ttggaaatagttctcggttgctctccacatggcgaggcac (SEQ ID NO: 13) |
| BIP: | tggtcaggtggagtaaaacataaggagacacctccatgctgtc (SEQ ID NO: 14) |
| LoopF: | caagaagaaaacaggttcctgg (SEQ ID NO: 15) |
| LoopB: | ggttcaaagcatgaaaaaaattgg (SEQ ID NO: 16) |
| Probe: | ccttcactttgagccaggcaatggtcag (SEQ ID NO: 17) |

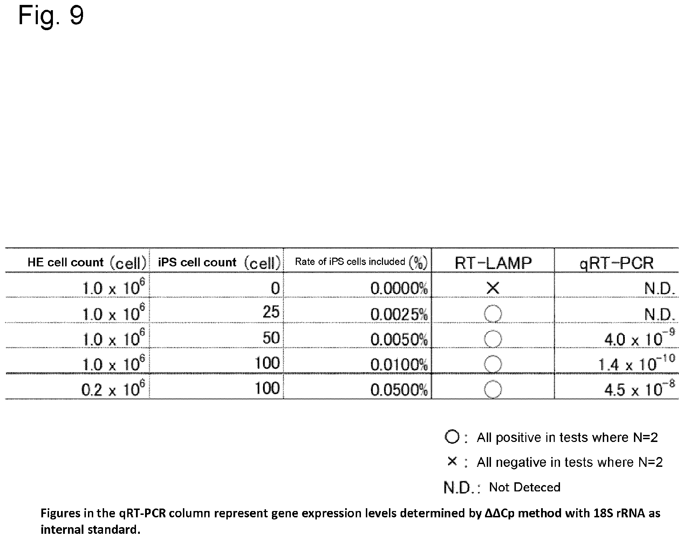

RT-LAMP was performed using samples prepared in the same manner as in Example 1. Briefly, iPS cells cultured under conditions for maintaining undifferentiated state were spiked gradually in iPS cell-derived hepatic progenitor cells (HE) to obtain samples. As a result, the expression of *LINC00678* was also detectable by RT-LAMP; further, RT-LAMP showed a higher sensitivity than qRT-PCR tested at the same time (Fig. 9).

### [Example 3]

Without directly detecting the expression of the marker gene of the present invention, residual undifferentiated cells can be detected if a reporter protein gene (e.g., fluorescent protein genes such as luciferase gene and GFP gene or genes such as *mouse CD4* that are expressed on cell surfaces and whose expression can be detected specifically as with antibody) is incorporated into the marker gene of the present invention under the control of the promoter region associated with regulation of its expression.

### [Example 4]

It becomes possible to select a highly safe undifferentiated cell clone from a plurality of iPS cell clones. Briefly, the expression of the marker gene of the present invention is evaluated in cells of interest as differentiated from the clones and cells that result from directed differentiation of the clones with a commercial kit such as a tri-lineage differentiation kit, and then those cell clones which are characterized by decreased expression of the marker gene are selected.

### [Example 5]

Differentiated cells are transplanted into a model animal over a long period of time, and engrafting cells are collected. By evaluating the expression of the marker gene of the present invention by qPCR, isothermal amplification (such as LAMP) or the like, or the method described in Example 3 above, undifferentiated cells in the formed tissue are detected.

### [Example 6]

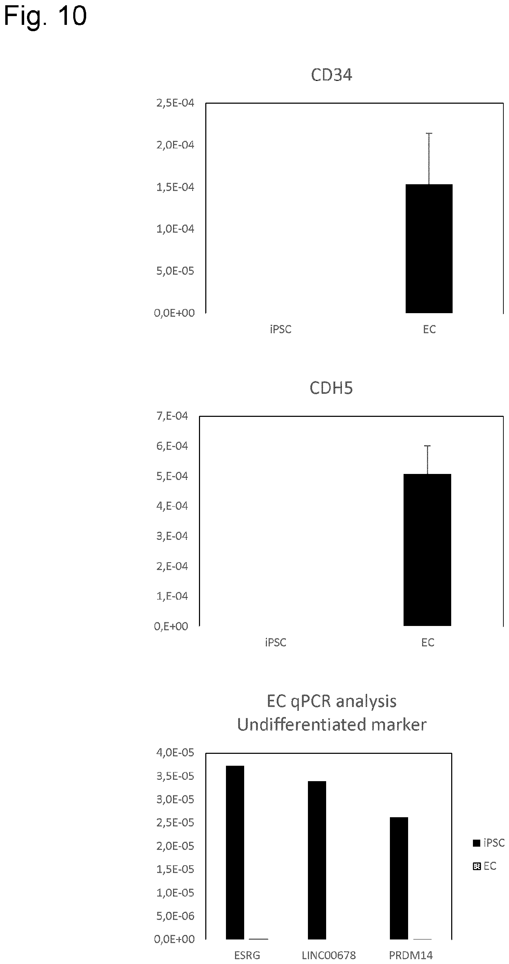

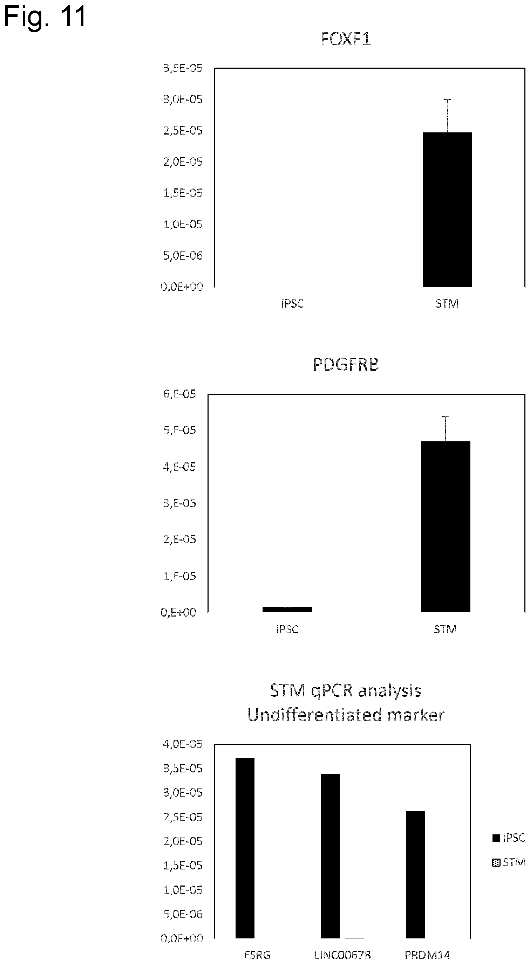

Residual undifferentiated iPS cells were tested in *septum transversum* mesenchyme cells (iPSC-STM/MC) (Cell Rep. 21:2661-2670, 2017) and vascular endothelial cells (iPSC-EC) (Cell Rep. 21:2661-2670 (2017)) as mesoderm-derived cells, both resulting from directed differentiation of iPS cells, in the same manner as in Example 1. Further, expression of marker genes therein was examined by qPCR. In either iPSC-STM/MC or iPSC-EC, *ESRG, LINC00678* and *PRDM14* decreased in differentiated cells without residual undifferentiated cells; therefore, it is believed that *ESRG, LINC00678* and *PRDM14* are useful as an undifferentiation marker for detecting residual undifferentiated cells (Figs. 10 and 11).

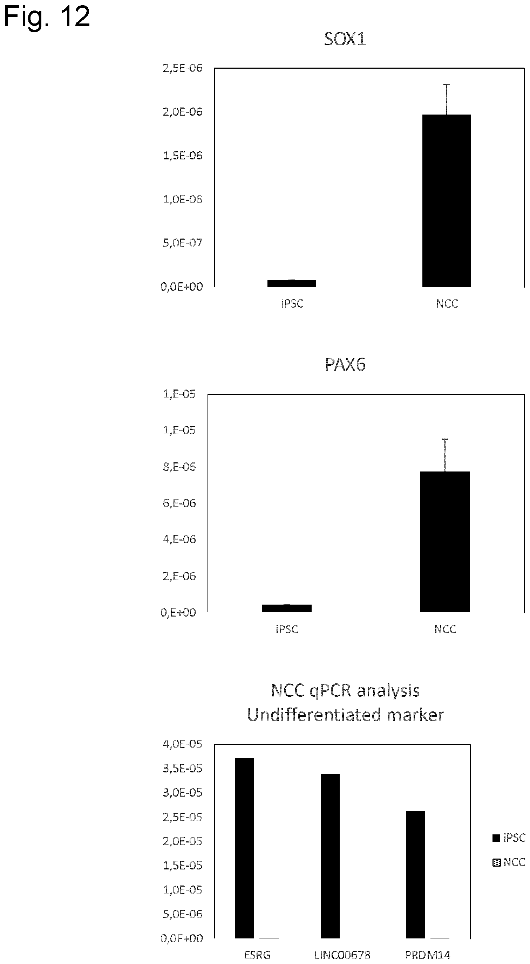

Further, similar results were obtained in measurement by qPCR using, as ectoderm-derived cells, neural crest cells (NCC) (Menendez L. et al., Proc Natl Acad Sci USA. 108(48):19240-5. 2011) resulting from directed differentiation of iPS cells (Fig. 12).

All publications, patents and patent applications cited herein are incorporated herein by reference in their entirety.

### INDUSTRIAL APPLICABILITY

The present invention is applicable to detection and evaluation of the undifferentiated cells that remain or become included in differentiated cells for use in regenerative medicine.

### SEQUENCE LISTING FREE TEXT

<SEQ ID NOS: 1 to 16>
   DNA sequences of primers are shown.
<SEQ ID NO: 17>
   DNA sequence of a probe is shown.

## Claims

1. A method of detecting undifferentiated cells, comprising measuring the expression level and/or the promoter activity of at least one gene selected from the group consisting of *LINC00678* and *PRDM14* in a differentiated cell population.

2. The method of claim 1, wherein the undifferentiated cell is embryonal carcinoma cell (EC cell), embryonic stem cell (ES cell), induced pluripotent stem cell (iPS cell) or embryonic germ cell (EG cell), and the differentiated cell population is a population of cells which have been differentiated from the undifferentiated cell.

3. The method of claim 1 or 2, wherein the differentiated cell population is a population of differentiated endodermal, mesodermal or ectodermal cells.

4. The method of claim 3, wherein the differentiated endodermal cells are hepatic endoderm cells.

5. The method of any one of claims 1 to 4, wherein the expression level of the gene is measured as the amount of RNA containing mRNA or the amount of protein.

6. The method of any one of claims 1 to 5, wherein the expression level of the gene is measured by qPCR, digital PCR, isothermal amplification of nucleic acids, immunostaining, *in situ* hybridization, RNA sequencing, microarray, NanoString, antibody array, flow cytometry or mass spectrometry.

7. A method of selecting a highly safe undifferentiated cell clone, comprising measuring the expression level and/or the promoter activity of at least one gene selected from the group consisting of *LINC00678* and *PRDM14* in differentiated endodermal, mesodermal or ectodermal cells that result from directed differentiation of an undifferentiated cell clone.

8. The method of claim 7, wherein the undifferentiated cell clone is an embryonal carcinoma cell (EC cell) clone, an embryonic stem cell (ES cell) clone, an induced pluripotent stem cell (iPS cell or iPSC) clone or an embryonic germ cell (EG cell) clone.

9. The method of claim 8, wherein the undifferentiated cell clone is an iPS cell clone.

10. A method of detecting undifferentiated cells, comprising measuring the expression level and/or the promoter activity of at least one gene selected from the group consisting of *LINC00678* and *PRDM14* in a tissue formed by transplanting differentiated cells into a model animal.

11. A method of using at least one gene selected from the group consisting of *LINC00678* and *PRDM14* as an undifferentiation marker for detecting undifferentiated cells in a differentiated cell population.

12. A kit for detecting undifferentiated cells, comprising a reagent capable of detecting the expression of at least one gene selected from the group consisting of *LINC00678* and *PRDM14* and/or a reagent capable of measuring the promoter activity of the gene.

13. The kit of claim 12, wherein the reagent capable of detecting the expression of the gene is primers, probes or antibodies.

14. The kit of claim 12, wherein the reagent capable of measuring the promoter activity of the gene is a gene sequence in which a reporter protein is ligated downstream of the promoter or a vector incorporating the gene sequence.


SEQUENCE LISTING

<110>  Pubilic University Corporation Yokohama City University
       EIKEN KAGAKU KABUSHIKI KAISHA

<120>  A method for detecting undifferentiated cells

<130>  FP-284PCT

<150>  JP2019-207002
<151>  2019-11-15

<160>  17

<170>  PatentIn version 3.5

<210>  1
<211>  25
<212>  DNA
<213>  Artificial Sequence

<220>
<223>  primer

<400>  1
catctcacca attttaaatc aggac                                          25


<210>  2
<211>  21
<212>  DNA
<213>  Artificial Sequence

<220>
<223>  primer

<400>  2
ctcccgtcat tctgctaaca c                                              21


<210>  3
<211>  19
<212>  DNA
<213>  Artificial Sequence

<220>
<223>  primer

<400>  3
gctcttggag gtggtgtcg                                                 19


<210>  4
<211>  19
<212>  DNA
<213>  Artificial Sequence

<220>
<223>  primer

<400>  4
gccggaaagg ttggaagtc                                                 19

<210>  5
<211>  18
<212>  DNA
<213>  Artificial Sequence

<220>
<223>  primer

<400>  5
tgcaccatgc gatttcag                                                    18


<210>  6
<211>  20
<212>  DNA
<213>  Artificial Sequence

<220>
<223>  primer

<400>  6
tgcatgaggc atagaccttc                                                  20


<210>  7
<211>  21
<212>  DNA
<213>  Artificial Sequence

<220>
<223>  primer

<400>  7
gacaattctg tgatgtggga g                                                21


<210>  8
<211>  21
<212>  DNA
<213>  Artificial Sequence

<220>
<223>  primer

<400>  8
tgacactgca cagcaactag g                                                21


<210>  9
<211>  18
<212>  DNA
<213>  Artificial Sequence

<220>
<223>  primer

<400>  9
ggcttcggat ccacattc                                                    18


<210>  10
<211>  20

<212>  DNA
<213>  Artificial Sequence

<220>
<223>  primer

<400>  10
agtggactcg catgtgtttg                                                    20


<210>  11
<211>  18
<212>  DNA
<213>  Artificial Sequence

<220>
<223>  primer

<400>  11
gacgggagtg tgagatcc                                                      18

<210>  12
<211>  22
<212>  DNA
<213>  Artificial Sequence

<220>
<223>  primer

<400>  12
acatcttctc ctgaatcctc ag                                                 22

<210>  13
<211>  40
<212>  DNA
<213>  Artificial Sequence

<220>
<223>  primer

<400>  13
ttggaaatag ttctcggttg ctctccacat ggcgaggcac                              40

<210>  14
<211>  43
<212>  DNA
<213>  Artificial Sequence

<220>
<223>  primer

<400>  14
tggtcaggtg gagtaaaaca taaggagaca cctccatgct gtc                          43

<210>  15
<211>  22
<212>  DNA
<213>  Artificial Sequence

<220>
<223>  primer

<400>  15

caagaagaaa acaggttcct gg 22

<210> 16
<211> 24
<212> DNA
<213> Artificial Sequence

<220>
<223> primer

<400> 16
ggttcaaagc atgaaaaaaa ttgg 24

<210> 17
<211> 28
<212> DNA
<213> Artificial Sequence

<220>
<223> probe

<400> 17
ccttcacttt gagccaggca atggtcag 28